# EUROPÄISCHE PATENTANMELDUNG

(11) **EP 1 205 465 A1**
(43) Veröffentlichungstag der Anmeldung: **15.05.2002**
(21) Anmeldenummer: 01125349.9
(22) Anmeldetag: 29.10.2001
(51) Int. Cl.: C07C 51/62, C07C 63/331, C07C 65/24

(54) **Verfahren zur Herstellung von gegebenenfalls substituierten Biphenyl-carbonsäurechloriden**

(30) Priorität: 09.11.2000 DE 10055498
(71) Anmelder: BAYER AG, 51368 Leverkusen (DE)
(72) Erfinder: Rodefeld, Lars, Dr., 51371 Leverkusen (DE); Klausener, Alexander, Dr., 50259 Pulheim (DE); Ullrich, Friedrich-Wilhelm, Dr., 51465 Bergisch Gladbach (DE)

(57) **Zusammenfassung**

Gegebenenfalls substituierte Biphenyl-carbonsäurechloride werden auf ökonomisch und ökologisch vorteilhafte Weise erhalten, wenn man Biphenyle mit Oxalylchlorid in einem Molverhältnis von Biphenyl zu Oxalylchlorid von 0,7 bis 1,5 und in Gegenwart einer Lewis-Säure umsetzt.

## Beschreibung

Die vorliegende Erfindung betrifft ein Verfahren zur Herstellung von gegebenenfalls substituierten Diphenyl-carbonsäurechloriden aus den entsprechenden Biphenylen und Oxalylchlorid.

Gegebenenfalls substituierte Biphenyl-carbonsäurechloride sind wichtige Zwischenprodukte zur Herstellung von Pflanzenschutz- und Pharma-Wirkstoffen (siehe z.B. EP-A 683 156).

Es sind schon Verfahren zur Herstellung von gegebenenfalls substituierten Biphenyl-carbonsäurechloriden bekannt. So kann man gegebenenfalls substituiertes Biphenyl acylieren, so ein Biphenylmethylketon erhalten, dieses zur entsprechenden Carbonsäure oxidieren und schließlich durch eine Chlorierung das gewünschte Biphenyl-carbonsäurechlorid erhalten (siehe Gazz. Chim. Ital. 79, 453 (1949)).

Man kann auch Biphenyle mit Chlorameisensäureamid zu Biphenyl-carbonsäureamiden umsetzen, diese zur entsprechenden Carbonsäure verseifen und schließlich auch hier nach Durchführung einer Chlorierung Biphenyl-carbonsäurechloride erhalten (siehe Angew. Chemie 61, 163 (1949)).

Gemäß einem dritten Weg wird zunächst eine Cyanbiphenyl-Verbindung hergestellt, die zur entsprechenden Säure verseift und letztere durch Chlorierung in das gewünschte Säurechlorid überführt wird (Synthesis 1991, 441 und CA 1958, 7233).

Nachteilig ist bei allen diesen Verfahren die Dreistufigkeit und der damit verbundene Einsatz von vielen Reaktanden und Hilfsstoffen. Nicht nur deren Bereitstellung, sondern auch die Entsorgung ihrer Folgeprodukte erfordern hohen Aufwand. Alle bekannte Verfahren zur Herstellung von Biphenyl-carbonsäurechloriden sind also in ökonomischer und ökologischer Hinsicht problematisch.

Es besteht deshalb immer noch ein Bedürfnis nach einem einfachen, kostengünstigen und ökologisch vorteilhaftem Verfahren zur Herstellung von Biphenyl-carbonsäurechloriden.

Umsetzungen von Aromaten und Diphenylverbindungen mit Oxalylchlorid sind auch schon beschrieben worden, wobei allerdings sehr verschiedenartige Reaktionsprodukte erhalten worden sind, beispielsweise Diarylethandione (siehe J. Org. Chem. 59, 635 (1994)), Diarylketone (siehe Tetrahedron Lett. 36, 5209 (1999)), Biscarbonsäurechloride (siehe Chem. Ber. 122, 2291 (1989)) und, ausgehend von Benzol und Oxalylchlorid, je nach Reaktionsführung, Benzoylchlorid oder Benzophenon (Ber. 41, 3566 (1908)).

Es ist auch beobachtet worden, dass man bei wässriger Aufarbeitung häufig nicht das Carbonsäurechlorid, sondern deren Verseifungsprodukt (die entsprechende Carbonsäure) erhält (siehe Org. Synth. Coll. Vol. V, 706 (1973)) und Friedel-Crafts and Related Reactions (III) S. 1259 (1964)).

Es liegen also sehr unübersichtliche Verhältnisse vor, und es ist unmöglich vorherzusehen, welche Reaktionsprodukte bei der Umsetzung von gegebenenfalls substituierten Biphenylen mit Oxalylchlorid zu erwarten sein könnten.

Es wurde nun ein Verfahren zur Herstellung von gegebenenfalls substituierten Bisphenyl-carbonsäurechloriden der Formel (I) gefunden, 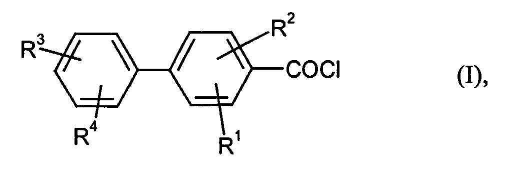 in der
R¹, R², R³ und R⁴ unabhängig voneinander jeweils für Wasserstoff, Halogen, C₁-C₅-Alkyl oder C₁-C₅-Alkoxy stehen,
das dadurch gekennzeichnet ist, dass man ein Biphenyl der Formel 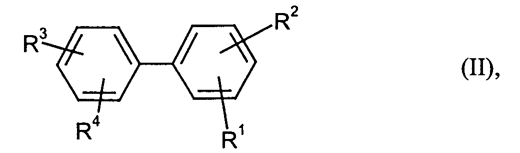 in der
R¹, R², R³ und R⁴ die bei Formel (I) angegebene Bedeutung haben,
mit Oxalylchlorid in einem Molverhältnis von Biphenyl der Formel (II) zu Oxalylchlorid von 0,7 bis 1,5 und in Gegenwart einer Lewis-Säure umsetzt.

In den Formeln (I) und (II) stehen die Reste R¹ bis R⁴ unabhängig voneinander vorzugsweise für Wasserstoff, Fluor, Chlor, Brom, Methyl, Ethyl, Methoxy oder Ethoxy.

Weiterhin stehen R¹ und R² bevorzugt für Wasserstoff und R³ und R⁴ bevorzugt für Wasserstoff, Fluor, Chlor, Brom, Methyl, Ethyl, Methoxy oder Ethoxy.

Weiterhin ist es bevorzugt, dass R¹, R² und R³ für Wasserstoff und R⁴ für Fluor, Chlor, Methyl, Ethyl, Methoxy oder Ethoxy in p-Stellung stehen.

Besonders bevorzugt stehen R¹, R² und R³ für Wasserstoff und R⁴ für Fluor oder Chlor in p-Stellung.

Biphenyle der Formel (II) sind auf vielerlei Weise oder analog zu bekannten Verfahren zugänglich und auch im Handel erhältlich (siehe z.B. Houben-Weyl, Band V/2b, 224 (1981), Org. Synth. Coll. Vol. V, 51 (1978), Chem. Ber. 95, 2469 (1995) und Synth. Comm. 29, 4423 (1999)).

Das Molverhältnis von Biphenyl der Formel (II) zu Oxalylchlorid beträgt vorzugsweise 0,9 bis 1,1. Besonders bevorzugt werden äquimolare Mengen eingesetzt.

Als Lewis-Säuren kommen für das erfindungsgemäße Verfahren z.B. die Chloride von Bor, Aluminium, Phosphor, Antimon, Eisen, Zink und Zinn in Frage. Bevorzugt ist Aluminiumchlorid.

Bezogen auf das Oxalylchlorid kann die Lewis-Säure z.B. in einem Molverhältnis von 0,9 bis 2,5 eingesetzt werden. Vorzugsweise liegt dieses Verhältnis bei 1 bis 1,5, insbesondere bei 1 bis 1,2.

Man kann das erfindungsgemäße Verfahren in Gegenwart eines Lösungsmittels durchführen. Geeignete Lösungsmittel sind z.B. mehrfach chlorierte Alkane wie Methylenchlorid, Dichlorethan und Tetrachlorethan und aromatische Verbindungen, die eine geringere Reaktivität mit Oxalylchlorid als das umzusetzende Biphenyl aufweisen, wie etwa Chlorbenzol oder o-Dichlorbenzol. Es ist natürlich darauf zu achten, dass nur solche Lösungsmittel eingesetzt werden, die unter den jeweiligen Reaktionsbedingungen flüssig sind.

Das erfindungsgemäße Verfahren kann beispielsweise bei Temperaturen im Bereich -30 bis +80°C durchgeführt werden. Bevorzugt sind Temperaturen im Bereich -20 bis +60°C, insbesondere solche im Bereich -10 bis +40°C.

Das erfindungsgemäße Verfahren kann man auf beliebige Weise durchführen. Beispielsweise kann man die Lewis-Säure, das Biphenyl der Formel (II) und das Lösungsmittel vorlegen und das Oxalylchlorid, gegebenenfalls zusammen mit weiterem Lösungsmittel, in die vorgelegte Mischung zudosieren. Man kann auch ein Gemisch aus Lewis-Säure und Lösungsmittel vorlegen und ein Gemisch aus Biphenyl der Formel (II) und Oxalylchlorid, gegebenenfalls zusammen mit weiterem Lösungsmittel, der Vorlage zudosieren. Man kann auch das Oxalylchlorid und eine Lösung des Biphenyls der Formel (II) im Lösungsmittel simultan zu der mit Lösungsmittel vorgelegten Lewis-Säure dosieren. Es sind noch weitere Durchführungsformen für das erfindungsgemäße Verfahren denkbar.

Nach Beendigung der Zusammengabe der Reaktanden, des Katalysators und gegebenenfalls des Lösungsmittels ist es im Allgemeinen vorteilhaft, noch einige Zeit bei einer Temperatur im Bereich -10 bis +40°C nachzurühren.

Die Aufarbeitung des nach der Reaktion und gegebenenfalls nach Ablauf der Nachrührzeit vorliegenden Gemisches kann beispielsweise so erfolgen, dass man das gesamte Reaktionsgemisch in ein Gemisch aus Eis und einer Mineralsäure dosiert, die wässrige Phase abtrennt und aus der organischen Phase das hergestellte Biphenyl-carbonsäurechlorid isoliert. Je nach den Ansprüchen an die Reinheit des hergestellten Biphenyl-carbonsäurechlorids kann gegebenenfalls zur weiteren Reinigung des Rohproduktes noch eine Kristallisation angeschlossen werden. Geeignete Lösungsmittel für eine derartige Kristallisation sind wenig polare bis unpolare Kohlenwasserstoffe. Beispielsweise kommen Alkane wie Hexan, Heptan, Octan, Nonan oder Decan inklusive aller möglicher Isomeren in Frage sowie Mischungen dieser Verbindungen.

Zur Aufarbeitung des Reaktionsgemisches ist es vorteilhaft in stark saurem Milieu zu arbeiten. Dann bleiben die aus der Lewis-Säure gebildeten Salze in Lösung. Als Mineralsäure ist Salzsäure bevorzugt. Wenn man ein frisch hergestelltes Gemisch von Eis und Salzsäure verwendet kann dessen Temperatur auch unter 0°C liegen. Im Laufe der Aufarbeitung steigt die Temperatur dann an. Vorzugsweise hält man die Temperatur des Aufarbeitungsgemisches, solange noch Wasser zugegen ist, im Bereich -20 bis +80°C, insbesondere im Bereich -5 bis +50°C.

Das erfindungsgemäße Verfahren hat die Vorteile, dass es in nur einer Reaktionsstufe und damit in ökonomisch und ökologisch vorteilhafter Weise Biphenyl-carbonsäurechloride in guten Ausbeuten und Reinheiten zugänglich macht.

### Beispiele

### Beispiel 1

In einem Rundkolben mit Rührer, Tropftrichter und Gasableitung, die über einen mit Aktivkohle gefüllten und mit Wasser betriebenen Waschturm geleitet wurde, wurden 350 ml o-Dichlorbenzol, 95,3 g p-Chlorbiphenyl und 70,7 g Aluminiumchlorid vorgelegt und auf 10°C gekühlt. In 30 min ließ man 70,2 g Oxalylchlorid zutropfen, wobei die Temperatur auf 10°C gehalten wurde. Nach beenderter Zugabe wurde der Ansatz 1 Stunde bei 10°C nachgerührt, dann 2 Stunden bei 40°C. Zur Aufarbeitung wurde der Ansatz in ein Gemisch aus 250 g Eis und 164 g 37 gew.-%ige wässrige Salzsäure unter Rühren eingetragen. Wenig ausgefallener Feststoff wurde durch Filtration entfernt. Die dabei anfallende Mutterlauge trennte sich in zwei Phasen. Aus der organischen Phase wurden nach Trocknung mit Natriumsulfat, Einengen und Kristallisation des Rückstandes aus Petrolether 77 % der Theorie 4-Chlorbiphenyl-4'-carbonsäurechlorid isoliert.

### Beispiel 2

In einem Rundkolben wie in Beispiel 1 verwendet, wurden 350 ml o-Dichlorbenzol, 95,3 g p-Chorbiphenyl und 64,7 g Oxalylchlorid vorgelegt und auf 0°C gekühlt. Im Verlaufe von 15 min ließ man eine gerührte Suspension von 70,7 g Aluminiumchlorid in 200 ml o-Dichlorbenzol zutropfen, wobei die Temperatur bei 0°C gehalten wurde. Nach beendeter Zugabe wurde der Ansatz 1 Stunde bei 0°C, dann eine weitere Stunde bei 20°C nachgerührt. Der Ansatz wurde in ein Gemisch aus 500 g Eis und 164 g 37 gew.-%iger wässrige Salzsäure unter Rühren eingetragen. Die analytische Untersuchung der sich ausbildenden organischen Phase ergab, dass diese 92 % der Theorie 4-Chlorbiphenyl-4'-carbonsäurechlorid enthielt.

### Beispiel 3

In einem Rundkolben wie in Beispiel 1 verwendet, wurden 200 ml o-Dichlorbenzol und 70,7 g Aluminiumchlorid vorgelegt und auf 0°C gekühlt. Bei dieser Temperatur wurde im Verlauf von 20 min eine Lösung von 95,3 g p-Chlorbiphenyl und 64,7 g Oxalylchlorid in 350 ml o-Dichlorbenzol zugetropft. Anschließend wurde der Ansatz 1 Stunde bei 0°C und eine weitere Stunde bei Raumtemperatur nachgerührt. Danach wurde das Reaktionsgemisch in eine Mischung aus 500 g Eis und 164 g 37 gew.-%iger wässriger Salzsäure unter Rühren eingegossen. Die sich bildende Wasserphase wurde abgetrennt und noch einmal mit 250 ml o-Dichlorbenzol gewaschen. Die organische Phase und die Waschflüssigkeit wurden vereinigt und das so erhaltene Gemisch einer Vakuumdistillation unterzogen. Zunächst ging ein Wasser-Dichlorbenzol-Gemisch über. Danach wurde die Destillation unterbrochen, um geringe Mengen entstandener Festprodukte abzufiltrieren. Anschließend wurde weiter destilliert, bis zu einer Sumpftemperatur von 120°C und einem Druck von 19 mbar. Die im Sumpf verbliebene Produktschmelze wurde auf 100°C abgekühlt und mit 250 ml Petrolether versetzt. Beim Abkühlen auf 4°C kristallisierte das gewünschte Produkt aus. Nach Absaugen und Trocknen wurden 118,9 g 4-Chlorbiphenyl-4'-carbonsäurechlorid mit einem Gehalt von 98,6 % erhalten. Das entspricht einer Ausbeute von 93 % der Theorie. Die Mutterlauge enthielt weitere 4 % der Theorie 4-Chlorbiphenyl-4'-carbonsäurechlorid.

### Beispiel 4

In einem Rundkolben wie in Beispiel 1 verwendet, wurden 200 ml o-Dichlorbenzol und 70,7 g Aluminiumchlorid vorgelegt und auf-5°C gekühlt. Bei dieser Temperatur wurden im Verlauf von 15 min 64,7 g Oxalylchlorid zudosiert. Zu dieser Suspension wurde eine Lösung von 95,3 g p-Chlordiphenyl in 350 ml o-Dichlorbenzol im Verlaufe von 1 Stunde bei einer Temperatur bis maximal +5°C getropft. Anschließend wurde der Ansatz 1 Stunde bei +5°C und eine weitere Stunde bei Raumtemperatur nachgerührt. Danach wurde das Reaktionsgemisch in ein Gemisch aus 500 g Eis und 164 g 37 gew.-%iger wässriger Salzsäure unter kräftigem Rühren eingegossen. Die Wasserphase wurde abgetrennt und mit 250 ml o-Dichlorbenzol gewaschen. Die organische Phase und die Waschflüssigkeit wurden vereinigt und dieses Gemisch durch Vakuumdestillation aufkonzentriert. Nach Kristallisation und Aufarbeitung entsprechend Beispiel 3 wurde 4-Chlor-diphenyl-4'-carbonsäurechlorid in einer Ausbeute von 93 % der Theorie erhalten.

## Patentansprüche

1. Verfahren zur Herstellung von gegebenenfalls substituierten Biphenyl-carbonsäurechloriden der Formel (I) 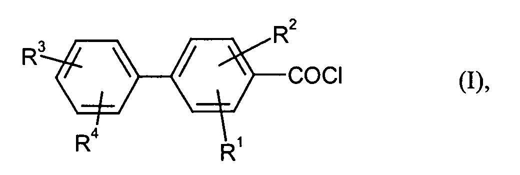 in der
R¹, R², R³ und R⁴ unabhängig voneinander jeweils für Wasserstoff, Halogen, C₁-C₅-Alkyl oder C₁-C₅-Alkoxy stehen,
**dadurch gekennzeichnet, dass** man ein Biphenyl der Formel 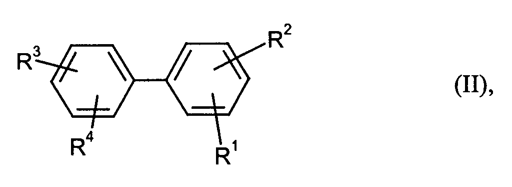 in der
R¹, R², R³ und R⁴ die bei Formel (I) angegebene Bedeutung haben,
mit Oxalylchlorid in einem Molverhältnis von Biphenyl der Formel (II) zu Oxalylchlorid von 0,7 bis 1,5 und in Gegenwart einer Lewis-Säure umsetzt.

2. Verfahren nach Anspruch 1, **dadurch gekennzeichnet, dass** in den Formeln (I) und (II) die Reste R¹ bis R⁴ unabhängig voneinander für Wasserstoff, Fluor, Chlor, Brom, Methyl, Ethyl, Methoxy oder Ethoxy stehen.

3. Verfahren nach Ansprüchen 1 und 2, **dadurch gekennzeichnet, dass** R¹, R² und R³ für Wasserstoff und R⁴ für Fluor, Chlor, Methyl, Ethyl, Methoxy oder Ethoxy in p-Stellung stehen.

4. Verfahren nach Ansprüchen 1 bis 3, **dadurch gekennzeichnet, dass** das Molverhältnis von Biphenyl der Formel (II) zu Oxalylchlorid 0,9 bis 1,1 beträgt.

5. Verfahren nach Ansprüchen 1 bis 4, **dadurch gekennzeichnet, dass** man als Lewis-Säure ein Chlorid von Bor, Aluminium, Phosphor, Antimon, Eisen, Zink oder Zinn einsetzt.

6. Verfahren nach Ansprüchen 1 bis 5, **dadurch gekennzeichnet, dass** auf 1 mol Oxalylchlorid 0,9 bis 2,5 mol Lewis-Säure eingesetzt werden.

7. Verfahren nach Ansprüchen 1 bis 6, **dadurch gekennzeichnet, dass** man es in Gegenwart eines Lösungsmittels durchführt.

8. Verfahren nach Ansprüchen 1 bis 7, **dadurch gekennzeichnet, dass** man es bei Temperaturen im Bereich -30 bis +80°C durchführt.

9. Verfahren nach Ansprüchen 1 bis 8, **dadurch gekennzeichnet, dass** man die Lewis-Säure und Lösungsmittel vorlegt und ein Gemisch aus Biphenyl der Formel (II) und Oxalylchlorid zudosiert.

10. Verfahren nach Ansprüchen 1 bis 9, **dadurch gekennzeichnet, dass** man das Reaktionsgemisch aufarbeitet, indem man es in ein Gemisch aus Eis und einer Mineralsäure dosiert, die wässrige Phase abtrennt und aus der organischen Phase das hergestellte Biphenyl-carbonsäurechlorid isoliert.
